# EUROPÄISCHE PATENTANMELDUNG

(11) **EP 4 810 472 A1**
(43) Veröffentlichungstag der Anmeldung: **23.09.2026**
(21) Anmeldenummer: 25164658.4
(22) Anmeldetag: 19.03.2025
(51) Int. Cl.: C07C 45/50, C07C 47/02, C07F 9/145, C07F 9/6574, C07F 15/00

(54) **LIGANDEN AUF BASIS VON MONOPHOSPHITEN UND MONOPHOSPHONITEN SOWIE DEREN VERWENDUNG**

(71) Anmelder: Evonik Oxeno GmbH & Co. KG, 45772 Marl (DE)
(72) Erfinder:
(74) Vertreter: Evonik Patent Association

(57) **Zusammenfassung**

Die vorliegende Erfindung betrifft das technische Gebiet der homogenen Katalyse. Insbesondere betrifft die vorliegende Erfindung Liganden auf Basis von Phosphor-(III)-Verbindungen sowie deren Herstellung und Verwendung in Hydroformylierungsreaktionen, sowie ein entsprechendes Verfahren zur Hydroformylierung.

## Beschreibung

Die vorliegende Erfindung betrifft das technische Gebiet der homogenen Katalyse. Insbesondere betrifft die vorliegende Erfindung Liganden auf Basis von Phosphor-(III)-Verbindungen sowie deren Herstellung und Verwendung in Hydroformylierungsreaktionen, sowie ein entsprechendes Verfahren zur Hydroformylierung.

Die vorliegende Erfindung betrifft speziell Verbindungen wie in den Ansprüchen 1 bis 4 definiert, ein Verfahren zu deren Herstellung sowie deren Verwendung und ein Verfahren zur Hydroformylierung unter Verwendung der beschriebenen Verbindungen.

Die Reaktion zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Die Produkte der Hydroformylierung werden vielfältig als Lösungsmittel oder als Zwischenprodukte für die Herstellung von Wasch- und Reinigungsmitteln, Schmiermitteln oder Weichmachern für Kunststoffe eingesetzt.

Durch den Einsatz von Katalysatorsystemen mit maßgeschneiderten Liganden, welche die Regio-, Stereo- und Enantioselektivität der Reaktion steuern können, wurde die Hydroformylierung ein wichtiges Werkzeug in der organischen Synthese von Feinchemikalien. In Hydroformylierungsreaktionen werden dabei häufig Verbindungen der Übergangsmetalle der VIII. Gruppe des Periodensystems der Elemente als Katalysatoren verwendet. Bekannte Liganden sind beispielsweise Verbindungen aus den Klassen der Phosphine, Phosphite und Phosphonite mit jeweils dreiwertigem Phosphor P(III). Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Beispielhafte Liganden, wie sie im Stand der Technik zum Einsatz kommen, sind etwa in der EP 2 947 090 A1 oder der EP 4 059 940 A1 beschrieben.

Eine unaufwändige Zugänglichkeit bzw. Verfügbarkeit und damit verbunden die gute Möglichkeit eines großtechnischen Einsatzes ist ein wichtiges Kriterium für Liganden. Ferner ist das Erreichen hoher Umsätze, gegebenenfalls in Kombination mit einer ausbalancierten n/iso-Selektivität (n/iso = Verhältnis von linearem Aldehyd (= n) zu verzweigtem (= iso) Aldehyd)), insbesondere für die großtechnisches Verwendung, von Bedeutung.

Die technische Aufgabe der Erfindung liegt also in der Bereitstellung neuer Verbindungen bzw. Liganden, welche insbesondere großtechnisch in der Hydroformylierung von Olefinen eingesetzt werden können und die Nachteile des Standes der Technik überwinden bzw. zumindest minimieren.

Insbesondere besteht eine Aufgabe der vorliegenden Erfindung darin, gegenüber bekannten Verbindungen bzw. Katalysatorsystemen des Standes der Technik eine gleichwertige oder sogar verbesserte Ausbeute zu liefern.

Gegenstand der vorliegenden Erfindung ist eine Verbindung nach Anspruch 1; weitere, vorteilhafte Ausgestaltungen dieses Erfindungsaspekts sind Gegenstand der diesbezüglichen Unteransprüche.

Weiterer Gegenstand der vorliegenden Erfindung - gemäß einem zweiten Aspekt der vorliegenden Erfindung - ist ein Verfahren nach Anspruch 6; weitere, vorteilhafte Ausgestaltungen dieses Erfindungsaspekts sind Gegenstand der diesbezüglichen Unteransprüche.

Wiederum weiterer Gegenstand der vorliegenden Erfindung - gemäß einem dritten Aspekt der vorliegenden Erfindung - ist eine katalytisch aktive Verbindung nach Anspruch 8; weitere, vorteilhafte Ausgestaltungen dieses Erfindungsaspekts sind Gegenstand der diesbezüglichen Unteransprüche.

Schließlich weitere Gegenstände der vorliegenden Erfindung - gemäß einem vierten und fünften Aspekt der vorliegenden Erfindung - sind ein Verfahren zur Hydroformylierung sowie eine diesbezügliche Verwendung; weitere, vorteilhafte Ausgestaltungen dieser Erfindungsaspekte sind Gegenstand der diesbezüglichen Unteransprüche.

Es versteht sich von selbst, dass Besonderheiten, Merkmale, Ausgestaltungen und Ausführungsformen sowie Vorteile oder dergleichen, welche nachfolgend - zu Zwecken der Vermeidung unnötiger Wiederholungen - nur zu einem Erfindungsaspekt ausgeführt werden, selbstverständlich in Bezug auf die übrigen Erfindungsaspekte entsprechend gelten, ohne dass es einer ausdrücklichen Erwähnung bedarf.

Weiterhin gilt, dass alle im Folgenden genannten Werte- bzw. Parameterangaben oder dergleichen grundsätzlich genormt bzw. standardisiert oder explizit angegebenen Bestimmungsverfahren oder mit dem Fachmann auf diesem Gebiet an sich geläufigen Bestimmungsmethoden ermittelt bzw. bestimmt werden können.

Darüber hinaus versteht es sich von selbst, dass alle gewichts- oder mengenbezogenen Prozentangaben vom Fachmann derart ausgewählt werden, dass in der Summe 100 % resultieren.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist eine Verbindung gemäß allgemeiner Formel (I) 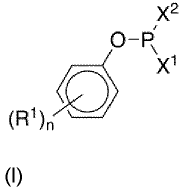 wobei,
- R¹, unabhängig voneinander, einen Propylrest, insbesondere *iso*-Propylrest, einen Butylrest, insbesondere *tert*-Butylrest, oder einen Methylrest bezeichnet, jedoch mit der Maßgabe, dass mindestens ein Rest R¹ einen Propylrest, insbesondere iso-Propylrest, oder einen Butylrest, insbesondere tert-Butylrest, bezeichnet,
- n eine ganze Zahl 1, 2 oder 3 darstellt,
- X¹ und X², gleich oder verschieden und/oder unabhängig voneinander, jeweils eine gegebenenfalls substituierte Phenylgruppe oder einen Rest O-R² bezeichnet, wobei R² einen organischen Rest, insbesondere aromatischen organischen Rest, bezeichnet, wobei X¹ und X² gegebenenfalls miteinander verbunden und/oder verbrückt sein können.

Bevorzugt ist dabei vorgesehen, dass R¹ ausgewählt ist aus einem der folgenden Reste: 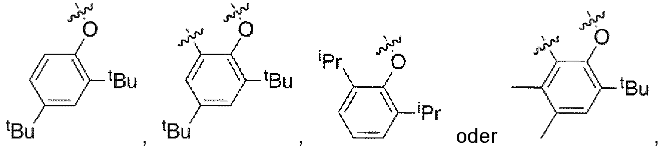 insbesondere

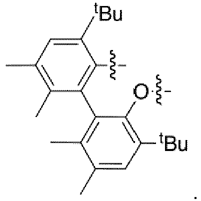

Für die Reste X¹ und X² wird es bevorzugt, wenn X¹ und X², gleich oder verschieden und/oder unabhängig voneinander, jeweils ausgewählt sind aus einem der folgenden Reste:

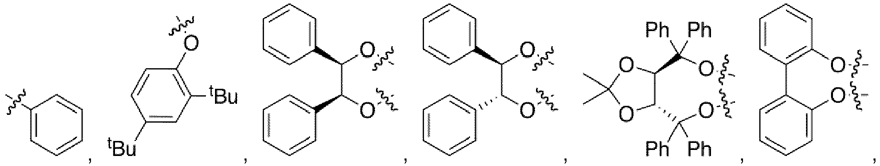

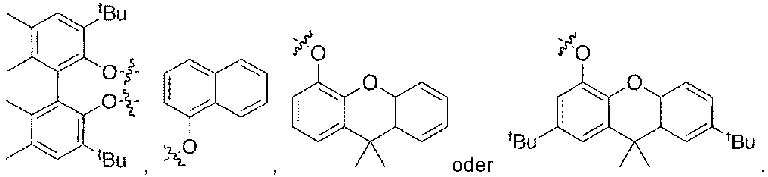

Für Verbindungen gemäß allgemeiner Formel (I), insbesondere wenn die Verbindungen Reste R¹, X¹ und X² wie zuvor vorzugsweise definiert aufweisen, zeichnen sich vorteilhaft durch eine vergleichsweise gute synthetische Zugänglichkeit aus.

Vorteilhaft können die Verbindungen aus kommerziell leicht verfügbaren bzw. synthetisch unaufwändig herstellbaren Ausgangsstoffen erhalten werden. Die ist speziell für großtechnische Einsätze von Vorteil.

Weiterhin hat sich vorteilhaft gezeigt, dass Verbindungen gemäß allgemeiner Formel (I), insbesondere wenn die Verbindungen Reste R¹, X¹ und X² wie zuvor vorzugsweise definiert aufweisen, konstant vergleichsweise hohe Ausbeute liefern. Darüber hinaus gestatten die Verbindungen eine zumindest im Wesentlichen weitestgehend vollständige Regenration des Katalysators, wenn die Verbindungen als Liganden in Hydroformylierungsreaktionen eingesetzt werden.

Im Rahmen der Erfindung ist es besonders bevorzugt, wenn die Verbindung gemäß Formel (I) aus einer der folgenden Verbindungen ausgewählt ist:

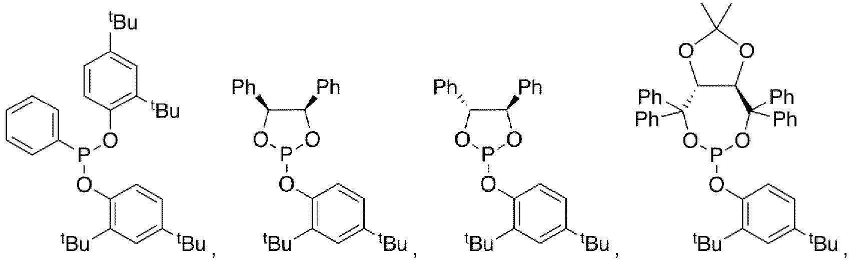

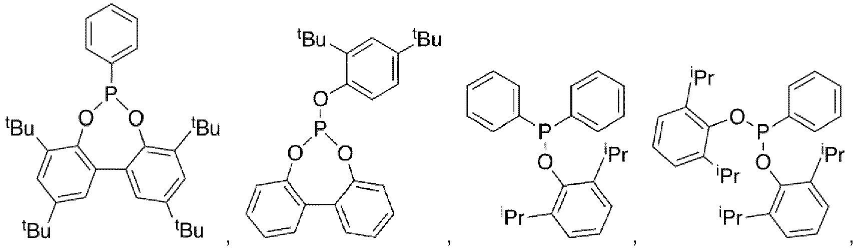

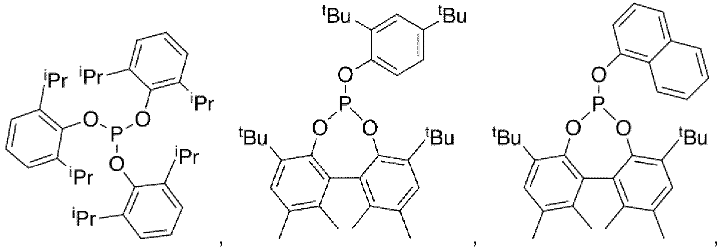

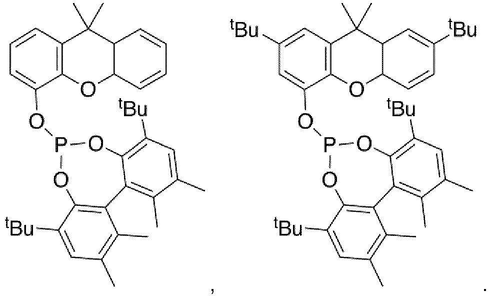

Für die vorgenannten bevorzugten Verbindungen werden die genannten Vorteile in besonderem Maße beobachtet.

Weiterer Gegenstand der vorliegenden Erfindung - gemäß einem **zweiten** Aspekt der vorliegenden Erfindung - ist ein Verfahren zur Herstellung einer Verbindung gemäß Formel (I), wobei eine reaktive Phosphor(III)spezies, insbesondere ausgewählt aus Phosphor(III)halogenid oder Phosphor(III)amidit, umgesetzt wird mit einem Hydroxylgruppen-haltigen Reagenz, so dass eine Verbindung gemäß Formel (I) resultiert.

Diesbezüglich hat es sich bewährt, wenn das Verfahren bei einer Temperatur im Bereich von 10 °C bis 120 °C und/oder in einem, bevorzugt organischen, insbesondere aprotischen, Lösemittel durchgeführt wird.

Weiter hat es sich bewährt, wenn das Verfahren in Gegenwart einer Base, insbesondere eines Amins, vorzugsweise Triethylamin, durchgeführt wird.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Wiederum weiterer Gegenstand der vorliegenden Erfindung - gemäß einem **dritten** Aspekt der vorliegenden Erfindung - ist eine katalytisch aktive Verbindung, wobei die katalytisch aktive Verbindung mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, zusammen mit mindestens einer Verbindung gemäß Formel (I), wie zuvor beschrieben, als Ligand umfasst.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Ebenfalls weitere Gegenstände der vorliegenden Erfindung - gemäß einem **vierten** Aspekt und einem **fünften** Aspekt der vorliegenden Erfindung - sind die Verwendung einer Verbindung gemäß Formel (I), wie zuvor beschrieben, oder einer katalytisch aktiven Verbindung, wie zuvor beschrieben, in einem Hydroformylierungsverfahren, insbesondere zur Umsetzung ethylenisch ungesättigter Verbindungen zu den entsprechenden Aldehyden, sowie ein Verfahren zur Hydroformylierung, wobei mindestens eine ethylenisch ungesättigte Verbindung in Gegenwart einer Verbindung gemäß Formel (I), wie zuvor beschrieben, oder einer katalytisch aktiven Verbindung, wie zuvor beschrieben, einer Hydroformylierung unterzogen wird, insbesondere zu dem entsprechenden Aldehyd umgesetzt wird.

Hierbei hat es sich sowohl für die Verwendung als auch das Verfahren nach der vorliegenden Erfindung bewährt, wenn das Hydroformylierungsverfahren die Verfahrensschritte:
a) Vorlegen einer ethylenisch ungesättigten Verbindung;
b) Zugabe einer Verbindung gemäß Formel (I), wie zuvor beschrieben, und einer Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, oder einer katalytisch aktiven Verbindung, wie zuvor beschrieben;
c) Zuführen von H₂ und CO,
d) Erwärmen des Reaktionsgemisches aus a) bis c), wobei das Olefin zu einem Aldehyd umgesetzt wird,
umfasst.

Weiter werden im Rahmen der Verwendung sowie des Verfahrens nach der vorliegenden Erfindung gute Ergebnisse erhalten, wenn die ethylenisch ungesättigte Verbindung in Verfahrensschritt a) ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

Auch hat es sich für die genannten Erfindungsaspekte bewährt, wenn die Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, ausgewählt ist aus: Rh(acac)(CO)₂, [(acac)Rh(COD)], wobei acac = Acetylacetonat-Anion und COD = 1,5-Cyclooctadien) sind, und/oder Rh₄CO₁₂, oder
wenn die katalytisch aktive Verbindung neben der mindestens einen Verbindung gemäß Formel (I) mindestens einen weiteren Liganden ausgewählt aus (acac), (CO) und/oder (COD) umfasst.

Vorzugsweise ist außerdem vorgesehen, dass das Zuführen von CO in Verfahrensschritt c) mit einem Druck in dem Bereich von 1 bis 6 MPa (10 bis 60 bar) erfolgt.

Ebenfalls ist es bevorzugt, wenn das Erwärmen des Reaktionsgemisches in Verfahrensschritt d) auf eine Temperatur in dem Bereich von 80 °C bis 160 °C erfolgt.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Der Gegenstand der vorliegenden Erfindung wird nachfolgend in nicht beschränkender Weise anhand der Ausführungsbeispiele erläutert.

### Ausführungsbeispiele:

Nachfolgend ist die Herstellung erfindungsgemäßer Verbindungen sowie deren Verwendung in einem Verfahren zur Hydroformylierung beschrieben.

### 1. Herstellung von Bis(2,4-di-tert-butylphenyl)phenylphosphonit (Verbindung 1)

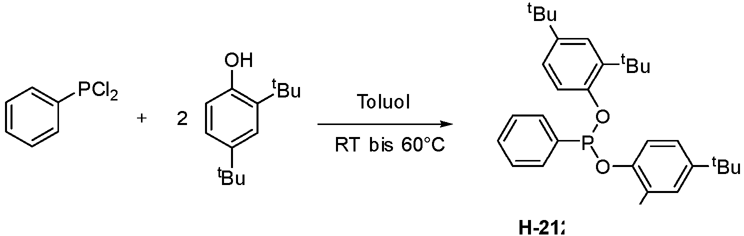

Ein Gemisch von 2,4-Di-tert-butylphenol (825 mg, 4 mmol) und Triethylamin (2.02 g, 20 mmol) werden bei Raumtemperatur gerührt und mit Dichlorphenylphosphin (358 mg, 2 mmol) tropfenweise versetzt. Nach etwa einer halben Stunde wird die Reaktionsmischung mit Toluol (2 ml) versetzt und bei 70 °C für 6 Stunden gerührt. Nach dem weiteren Rühren über Nacht bei Raumtemperatur wird weiteres Toluol (5 ml) hinzugegeben und der gesamte Ansatz auf Eis mit ca. 1.5 ml konzentrierter Salzsäure. Es wird intensiv gerührt bis zum vollständigen Schmelzen des Eises und die wässrige Lösung wird anschließend mit Toluol (3×10 ml) extrahiert. Die vereinigten organischen Phasen werden mit NaHCO₃-Lösung und Wasser gewaschen, mit Na₂SO₄ getrocknet und eingeengt. Nach Umkristallisation des Rohproduktes aus Acetonitril wird das Phosphonit erhalten.

Ausbeute: 695 mg weißer Feststoff (67%).

³¹P-NMR (CDCl₃): d +154.9 ppm.

¹H-NMR (CDCl₃): d 7.95 (2H, m, 2xHₐᵣ), 7.52-7.47 (3H, m, 3xHₐᵣ), 7.34 (2H, d, *J* 2.5 Hz, 2xHₐᵣ), 7.15 (2H, dd, *J* 8.4, 2.7 Hz, 2xHₐᵣ), 7.09 (2H, dd, *J* 8.4, 2.4 Hz, 2xHₐᵣ), 1.33 (18H, s, 2xC(CH₃)₃), 1.31 (18H, s, 2xC(CH₃)₃).

¹³C-NMR (CDCl₃): d 151.9 (d, *J* 7.6 Hz, 2xCₐᵣ-O), 145.0 (2xCₐᵣ), 140.5 (d, *J* 11.8 Hz, Cₐᵣ-P), 138.6 (d, *J* 2.6 Hz, 2xCₐᵣ), 131.0 (CₐᵣH), 130.5 (d, *J* 28.1 Hz, 2xCₐᵣH), 128.3 (d, *J* 8.5 Hz, 2xCₐᵣH), 124.1 (2xCₐᵣH), 123.5 (2xCₐᵣH), 117.9 (d, *J* 20.5 Hz, 2xCₐᵣH), 34.9 (2x^{t}C), 34.4 (2x^{t}C), 31.6 (6xCH₃), 31.0 (6xCH₃).

HRMS (EI) [M]⁺: m/z berechnet: für C₃₄H₄₇O₂P 518.33082, gefunden: 518.32967.

### 2. Herstellung von (4S,5R)-2-(2,4-Di-tert-butylphenoxy)-4,5-diphenyl-1,3,2-dioxaphospholan (Verbindung 2)

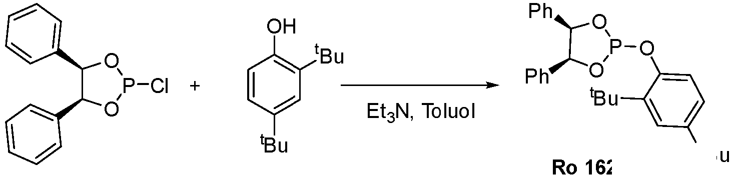

Eine Lösung mit 2,4-Di-tert-butylphenol (246 mg, 1.19 mmol) und Triethylamin (181 mg, 1.79 mmol) in Toluol (2 ml) wird bei 0 °C vorgelegt und anschließend langsam mit (4*R*,5*S*)-2-Chloro-4,5-diphenyl-1,3,2-dioxaphospholan (359 mg, 1.29 mmol) in Toluol (2 ml) versetzt. Nach dem Rühren über Nacht bei Raumtemperatur wird vom gebildeten Hydrochlorid abfiltriert und anschließend das Lösemittel unter Vakuum entfernt. Der verbleibende feste Rückstand wird unter Vakuum getrocknet und musste nicht weiter gereinigt werden. Das Produkt fiel als ein Gemisch von *cis-* und *trans-*Isomeren an.

Ausbeute: 345 mg weißer Feststoff (44%).

³¹P-NMR (CD₂Cl₂): d +141.7 ppm (21%, cis-Verbindung) und +132.3 ppm (79%, trans-Verbindung).

¹H-NMR (CD₂Cl₂), *cis-Isomer:* d 7.54 (1H, d, *J* 2.5 Hz, Hₐᵣ), 7.33-7.00 (12H, m, 12xHₐᵣ), 5.81 (2H, d, *J* 8.6 Hz, 2xCH-O), 1.56 (9H, s, C(CH₃)₃), 1.40 (9H, s, C(CH₃)₃); trans-Isomer: d 7.50 (1H, d, *J* 2.4 Hz, Hₐᵣ), 7.33-7.00 (12H, m, 12xHₐᵣ), 5.95 (2H, d, *J* 1.6 Hz, 2xCH-O), 1.52 (9H, s, C(CH₃)₃), 1.39 (9H, s, C(CH₃)₃).

¹³C-NMR (CD₂Cl₂), *cis-Isomer:* d 148.4 (d, *J* 9.2 Hz, Cₐᵣ-O), 147.2 (Cₐᵣ), 140.7 (d, *J* 2.6 Hz, Cₐᵣ), 137.2 (Cₐᵣ), 128.2 (4xCₐᵣH), 128.1 (4xCₐᵣH), 127.9 (2xCₐᵣH), 125.1 (CₐᵣH), 124.2 (CₐᵣH), 121.2 (d, *J* 16.3 Hz, CₐᵣH), 83.7 (d, *J* 9.6 Hz, CH-O), 35.3 (^{t}C), 35.0 (^{t}C), 31.8 (3xCH₃), 30.5 (3xCH₃); *trans-Isomer:* d 149.1 (d, *J* 7.5 Hz, Cₐᵣ-O), 146.7 (Cₐᵣ), 140.1 (d, *J* 2.3 Hz, Cₐᵣ), 136.4 (d, *J* 4.0 Hz, Cₐᵣ), 128.2 (4xCₐᵣH), 128.2 (2xCₐᵣH), 127.4 (4xCₐᵣH), 125.0 (CₐᵣH), 124.2 (CₐᵣH), 120.2 (d, *J* 15.8 Hz, CₐᵣH), 82.1 (d, *J* 6.6 Hz, CH-O), 35.3 (^{t}C), 34.9 (^{t}C), 31.8 (3xCH₃), 30.5 (3xCH₃).

HRMS (ESI-TOF) [M+H]⁺: m/z berechnet: für C₂₈H₃₃O₃P 449.2245, gefunden: 449.2251.

| | | | | |
|---|---|---|---|---|
| Elementaranalyse: | berechnet: | C 74.98% | H 7.41% | P 6.91% |
| | gefunden: | C 74.96% | H 7.38% | P 6.95% |

### 3. Herstellung von (4R,5R)-2-(2,4-Di-tert-butylphenoxy)-4,5-diphenyl-1,3,2-dioxaphospholan (Verbindung 3)

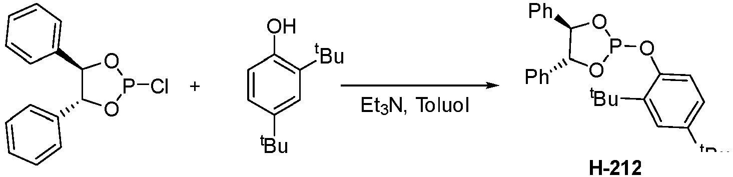

Eine Lösung mit 2,4-Di-*tert*-butylphenol (202 mg, 2.00 mmol) und Triethylamin (304 mg, 3.00 mmol) in Toluol (4 ml) wird bei 0 °C vorgelegt und anschließend langsam mit (4*R*,5*S*)-2-Chloro-4,5-diphenyl-1,3,2-dioxaphospholan (454 mg, 2.1 mmol) in Toluol (4 ml) versetzt. Nach dem Rühren über Nacht bei Raumtemperatur wird vom gebildeten Hydrochlorid abfiltriert und anschließend das Lösemittel unter Vakuum entfernt. Der verbleibende feste Rückstand wird unter Vakuum getrocknet und anschließend durch Säulenchromatographie (Eluent: Cyclohexan/AcOEt 99/1) von HydrolyseProdukten und restlichen Phenol gereinigt.

Ausbeute: 200 mg weißer Feststoff (22%).

³¹P-NMR (CDCl₃): d +134.7 ppm.

¹H-NMR (CDCl₃): d 7.51 (1H, d, *J* 2.3 Hz, Hₐᵣ), 7.45-7.32 (10H, m, 10xHₐᵣ), 7.25 (1H, dd, *J* 8.3 2.4 Hz, Hₐᵣ), 7.23 (1H, d, *J* 8.3 Hz, Hₐᵣ), 5.40 (1H, d, *J* 9.5 Hz, CH-O), 5.03 (1H, dd, *J* 9.5, 2.6 Hz, CH-O), 1.56 (9H, s, C(CH₃)₃), 1.40 (9H, s, C(CH₃)₃).

¹³C-NMR (CDCl₃): d 148.5 (d, *J* 8.2 Hz, Cₐᵣ-O), 146.2 (Cₐᵣ), 139.7 (d, *J* 2.0 Hz, Cₐᵣ), 136.6 (Cₐᵣ), 135.9 (d, *J* 5.8 Hz, Cₐᵣ), 128.8 (CₐᵣH), 128.7 (2xCₐᵣH), 128.6 (2xCₐᵣH), 128.5 (CₐᵣH), 127.4 (2xCₐᵣH), 126.9 (2xCₐᵣH), 124.5 (CₐᵣH), 123.7 (CₐᵣH), 120.1 (d, *J* 15.9 Hz, CₐᵣH), 87.4 (d, *J* 7.9 Hz, CH-O), 84.0 (d, *J* 6.7 Hz, CH-O), 35.0 (^{t}C), 34.5 (^{t}C), 31.6 (3xCH₃), 30.2 (3xCH₃).

### 4. Herstellung von (3aR,8aR)-6-(2,4-Di-tert-butylphenoxy)-2,2-dimethyl-4,4,8,8-tetraphenyltetrahydro-[1,3]dioxolo[4,5-e][1,3,2]dioxaphosphepin (Verbindung 4)

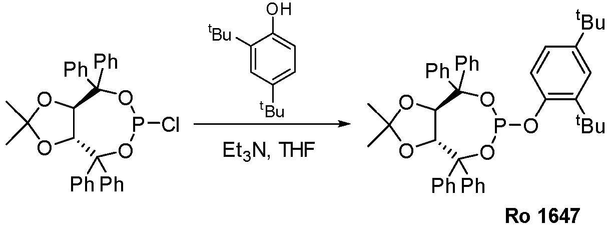

Zu einer Lösung des 2,4-Di-*tert*-butylphenols (122 mg, 0.59 mmol) und Triethylamins (90 mg, 0.89 mmol) in THF (1 ml) gibt man bei 0 °C tropfenweise eine Lösung des (3aR,8aR)-6-Chloro-2,2-dimethyl-4,4,8,8-tetraphenyltetrahydro-[1,3]dioxolo[4,5-*e*][1,3,2]dioxaphosphepins (315 mg, 0.59 mmol) in THF (3 ml). Nach dem Rühren über Nacht bei Raumtemperatur wird vom ausgefallenem Hydrochlorid abfiltriert und das Filtrat unter Vakuum eingeengt. Das Produkt wurde durch Säulenchromatographie (Eluent: Heptan/Dichlormethan 1/1) gereinigt.

Ausbeute: 245 mg weißer Feststoff (59%).

³¹P-NMR (CD₂Cl₂): d +138.8 ppm.

¹H-NMR (CDCl₃): d 7.69-7.20 (21H, m, 21xHₐᵣ), 7.11 (1H, dd, *J* 8.3, 2.4 Hz, Hₐᵣ), 7.05 (1H, dd, *J* 8.3, 1.5 Hz, Hₐᵣ), 5.37 (1H, d, *J* 8.2 Hz, CH-O), 5.32 (1H, dd, *J* 8.2, 1.4 Hz, CH-O), 1.37 (9H, s, C(CH₃)₃), 1.31 (9H, s, C(CH₃)₃), 0.98 (3H, s, CH₃), 0.47 (3H, s, CH₃).

¹³C-NMR (CDCl₃): d 149.5 (d, *J* 9.7 Hz, Cₐᵣ-O), 146.1 (2xCₐᵣ), 145.6 (d, *J* 1.4 Hz, Cₐᵣ), 142.2 (d, *J* 3.7 Hz, Cₐᵣ), 141.4 (d, *J* 1.4 Hz, Cₐᵣ), 139.5 (d, *J* 2.7 Hz, Cₐᵣ), 129.3 (2xCₐᵣH), 129.1 (CₐᵣH), 129.1 (CₐᵣH), 128.8 (2xCₐᵣH), 128.5 (CₐᵣH), 128.2 (2xCₐᵣH), 127.9 (CₐᵣH), 127.9 (CₐᵣH), 127.7-127.5 (9xCₐᵣH), 124.6 (CₐᵣH), 123.7 (CₐᵣH), 119.6 (d, *J* 18.0 Hz, CₐᵣH) 114.1 (^{t}CMe₂), 86.7 (d, *J* 8.2 Hz, ^{t}C-O), 83.9 (d, *J* 2.1 Hz, ^{t}C-O), 82.3 (d, *J* 14.0 Hz, CH-O), 81.4 (d, *J* 4.2 Hz, C-O), 35.2 (^{t}C), 34.8 (^{t}C), 31.7 (3xCH₃), 31.2 (3xCH₃), 27.3 (CH₃), 26.2 (CH₃).

HRMS (ESI-TOF) [M+Na]⁺: m/z berechnet: für C₄₅H₄₉O₅P 723.3215, gefunden: 723.3227.

| | | | | |
|---|---|---|---|---|
| Elementaranalyse: | berechnet: | C 77.12% | H 7.05% | P 4.42% |
| | gefunden: | C 77.07% | H 7.15% | P 4.40% |

### 5. Herstellung von 2,4,8,10-Tetra-tert-butyl-6-phenyldibenzo[d,f][1,3,2]dioxaphosphepin (Verbindung 5)

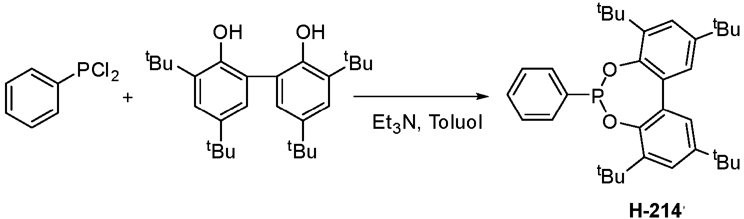

Ein Gemisch von 3,3',5,5'-Tetra-tert-butyl-[1,1'-biphenyl]-2,2'-diol (411 mg, 1 mmol) und Triethylamin (205 mg, 2.03 mmol) in Toluol (3 ml) werden bei Raumtemperatur gerührt und mit Dichlorphenylphosphin (179 mg, 1 mmol) tropfenweise versetzt. Anschließend wird die Reaktionsmischung bei 70 °C etwa 16 Stunden gerührt. Nach dem Abkühlen wird vom ausgefallenen Hydrochlorid abfiltriert und das Filtrat unter Vakuum eingeengt. Der erhaltene feste Rückstand wird abschließend aus Acetonitril umkristallisiert.

Ausbeute: 375 mg weißer Feststoff (73%), Smp. = 168-169 °C.

³¹P-NMR (CD₂Cl₂): d +181.1 ppm.

¹H-NMR (CD₂Cl₂): d 7.53-7.43 (3H, m, 3xHₐᵣ), 7.39 (2H, d, *J* 2.5 Hz, 2xHₐᵣ), 7.35-7.28 (2H, m, 2xHₐᵣ), 7.21 (2H, dd, *J* 2.5, 0.4 Hz, 2xHₐᵣ), 1.38 (18H, s, 2xC(CH₃)₃), 1.18 (18H, s, 2xC(CH₃)₃) ppm.

¹³C-NMR (CD₂Cl₂): d 147.3 (d, *J* 5.7 Hz, 2xCₐᵣ-O), 147.2 (2xCₐᵣ), 141.0 (d, *J* 36.4 Hz, Cₐᵣ-P), 140.5 (d, *J* 1.3 Hz, 2xCₐᵣ), 133.8 (d, *J* 3.9 Hz, 2xCₐᵣ), 132.6 (CₐᵣH), 131.8 (d, *J* 29.8 Hz, 2xCₐᵣH), 128.4 (d, *J* 8.3 Hz, 2xCₐᵣH), 126.8 (2xCₐᵣH), 124.9 (2xCₐᵣH), 35.6 (2x^{t}C), 35.0 (2x^{t}C), 31.7 (6xCH₃), 31.2 (d, *J* 2.5 Hz, 6xCH₃).

### 6. Herstellung von 6-(2,4-Di-tert-butylphenoxy)dibenzo[d,f][1,3,2]dioxaphosphepin (Verbindung 6)

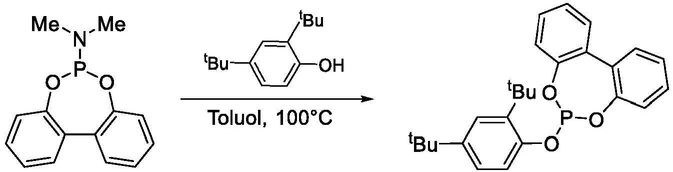

Eine Lösung von N,N-Dimethyldibenzo[*d,f*][1,3,2]dioxaphosphepin-6-amin (200 mg, 0.77 mmol) und 2,4-Di-*tert*-butylphenols (159 mg, 0.77 mmol) werden in Toluol (4 ml) für zwei Tage bei 100 °C gerührt. Ein leichter Argon-Strom bewirkt, dass das freigesetzte Dimethylamin aus dem Reaktionsansatz ausgetrieben wird. Nach dieser Zeit lässt sich nach dem Abkühlen im ³¹P-NMR kein Edukt mehr nachweisen und zur Aufarbeitung wird das Lösemittel unter Vakuum entfernt.

Aus dem Rückstand wird durch eine rasche Säulenchromatographie (Eluent: Cyclohexan/AcOEt 99/1) das Produkt als weißer Feststoff isoliert.

Ausbeute: 140 mg weißer Feststoff (43%), Smp. = 50-52 °C.

³¹P-NMR (CD₂Cl₂): d +145.2 ppm.

¹H-NMR (CD₂Cl₂): d 7.57 (2H, dd, *J* 7.5, 1.9 Hz, 2xHₐᵣ), 7.48 (1H, m, Hₐᵣ), 7.46-7.30 (6H, m, 6xHₐᵣ), 7.27 (2H, br d, *J* 1.4 Hz, 2xHₐᵣ), 1.45 (9H, s, C(CH₃)₃), 1.38 (9H, s, C(CH₃)₃).

¹³C-NMR (CD₂Cl₂): d 149.7 (d, *J* 5.4 Hz, 2xCₐᵣ-O), 148.9 (d, *J* 8.8 Hz, Cₐᵣ-O), 147.1 (Cₐᵣ), 140.0 (d, *J* 2.9 Hz, Cₐᵣ), 131.7 (d, *J* 3.1 Hz, 2xCₐᵣ), 130.5 (2xCₐᵣH), 129.7 (2xCₐᵣH), 125.9 (2xCₐᵣH), 125.1 (CₐᵣH), 124.3 (CₐᵣH), 122.5 (2xCₐᵣH), 119.9 (d, *J* 15.4 Hz, CₐᵣH), 35.3 (^{t}C), 34.9 (^{t}C), 31.7 (3xCH₃), 30.3 (3xCH₃).

HRMS (EI) [M]⁺: m/z berechnet: für C₂₆H₂₉O₃P 420.18488, gefunden: 420.18597.

### 7. Herstellung von (2,6-Diisopropylphenoxy)diphenylphosphin (Verbindung 7)

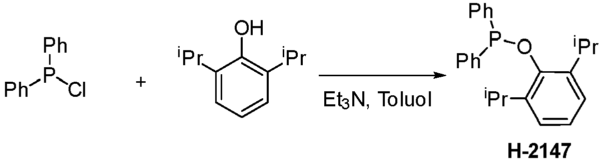

Ein Gemisch von 2,6-Diisopropylphenol (713 mg, 4 mmol) und Triethylamin (810 mg, 8 mmol) werden bei Raumtemperatur gerührt und mit Chlordiphenylphosphin (883 mg, 4 mmol) tropfenweise versetzt. Nach etwa einer halben Stunde wird die Reaktionsmischung mit Toluol (4 ml) versetzt und bei 70 °C für 20 Stunden gerührt. Zur Aufarbeitung wird vom Niederschlag abfiltriert und einmal mit Toluol (5 ml) nachgewaschen. Entfernen des Lösemittels unter Vakuum und Trocknen bei 40 °C liefert ein Rohprodukt, dass nicht weiter gereinigt werden muss. Zur Verbesserung der Reinheit lässt sich das Rohprodukt aus Hexan umkristallisieren.

Ausbeute: 820 mg weißer Feststoff (57%), Smp. 82-85 °C.

³¹P-NMR (CD₂Cl₂): d +116.8 ppm.

¹H-NMR (CD₂Cl₂): d 7.74-7.65 (4H, m, 4xHₐᵣ), 7.47-7.43 (6H, m, 6xHₐᵣ), 7.10-7.04 (3H, m, 3xHₐᵣ), 3.02 (2H, dsept, *J* 6.8,1.5 Hz, 2xCH), 1.00 (12H, d, *J* 6.9 Hz, 4xCH₃).

¹³C-NMR (CD₂Cl₂): d 152.5 (d, *J* 6.7 Hz, Cₐᵣ-O), 142.7 (d, *J* 19.2 Hz, 2xCₐᵣ-P), 141.3 (d, *J* 3.2 Hz, 2xCₐᵣ), 131.0 (d, *J* 23.9 Hz, 4xCₐᵣH), 130.1 (2xCₐᵣH), 128.8 (d, *J* 7.5 Hz, 4xCₐᵣH), 124.5 (d, *J* 1.7 Hz, CₐᵣH), 124.3 (d, *J* 1.3 Hz, 2xCₐᵣH), 27.5 (d, *J* 3.8 Hz, 2xCH), 23.7 (4xCH₃).

HRMS (EI) [M]⁺: m/z berechnet: für C₂₄H₂₇OP 362.17940, gefunden: 362.18008.

### 8. Herstellung von Bis(2,6-diisopropylphenyl)phenylphosphonit (Verbindung 8)

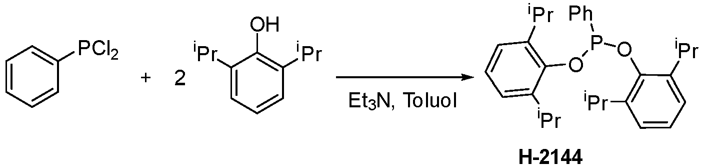

Ein Gemisch von 2,6-Diisopropylphenol (713 mg, 4 mmol) und Triethylamin (2.02 g, 20 mmol) werden bei Raumtemperatur gerührt und mit Dichlorphenylphosphin (358 mg, 2 mmol) tropfenweise versetzt. Nach etwa einer halben Stunde wird die Reaktionsmischung mit Toluol (2 ml) versetzt und zunächst bei 70 °C für 4 Stunden gerührt. Nach dem Rühren über Nacht bei Raumtemperatur wird weiteres Toluol (5 ml) zugegeben und der gesamte Ansatz auf Eis mit ca. 1.5 ml konzentrierter Salzsäure gegossen. Es wird intensiv gerührt bis zum vollständigen Schmelzen des Eises und die wässrige Lösung wird anschließend mit Toluol (3x10 ml) extrahiert. Die vereinigten organischen Phasen werden mit NaHCO₃-Lösung und Wasser gewaschen, getrocknet mit Na₂SO₄ und eingeengt. Nach Umkristallisation des Rohproduktes mit geringen Mengen Hexan und Aufbewahren in der Kälte wird das Phosphonit erhalten.

Ausbeute: 680 mg weißer Feststoff (73%), Smp. 84-86 °C.

³¹P-NMR (CD₂Cl₂): d +174.9 ppm.

¹H-NMR (CD₂Cl₂): d 7.98-7.90 (2H, m, 2xHₐᵣ), 7.58-7.47 (3H, m, 3xHₐᵣ), 7.09-7.06 (6H, m, 6xHₐᵣ), 3.23 (4H, dspept, *J* 6.9, 1.8 Hz, 4xCH), 1.05 (12H, d, *J* 6.9 Hz, 4xCH₃), 1.02 (12H, d, *J* 6.9 Hz, 4xCH₃).

¹³C-NMR (CD₂Cl₂): d 149.2 (d, *J* 2.6 Hz, 2xCₐᵣ-O), 142.9 (d, *J* 23.4 Hz, Cₐᵣ-P), 141.2 (d, *J* 2.8 Hz, 4xCₐᵣ), 131.9 (CₐᵣH), 130.5 (d, *J* 28.5 Hz, 2xCₐᵣH), 128.8 (d, *J* 8.4 Hz, 2xCₐᵣH), 124.8 (d, *J* 1.6 Hz, 2xCₐᵣH), 124.3 (d, *J* 1.3 Hz, 4xCₐᵣH), 27.7 (d, *J* 4.9 Hz, 4xCH), 23.8 (4xCH₃), 23.7 (4xCH₃).

HRMS (EI) [M]⁺: m/z berechnet: für C₃₀H₃₉O₂P 462.26822, gefunden: 462.26753.

### 9. Herstellung von Tris(2,6-diisopropylphenyl)phosphit (Verbindung 9)

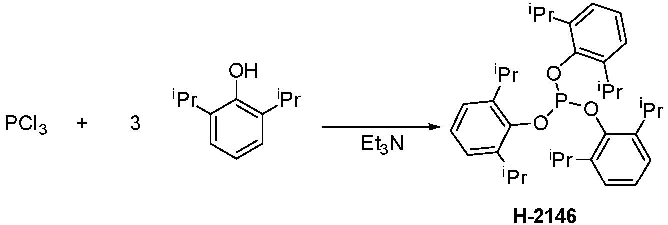

2,6-Diisopropylphenol (891 mg, 5 mmol) und Phosphortrichlorid (215 mg, 1.58 mmol) in Dichlormethan (8 ml) werden bei Raumtemperatur langsam mit einer Lösung von Triethylamin (700 mg, 6.90 mmol) in Dichlormethan (2 ml) versetzt. Nach ca. zwei Tagen entfernt man das Dichlormethan unter Vakuum und versetzt den verbleibenden weißen Rückstand mit Hexan (4 ml). Nach der Filtration wäscht man mit Hexan (2x 1.5 ml) nach und reduziert das Volumen (-4-5 ml). Während des Stehenlassens im Kühlschrank fällt das Produkt in Form weißer Nadeln aus.

Ausbeute: 410 mg weißer Feststoff (46%), Smp. 250-252 °C.

³¹P-NMR (CD₂Cl₂): d +146.8 ppm.

¹H-NMR (CD₂Cl₂): d 7.12-7.09 (9H, m, 9x arom. H), 3.45 (2H, dsept, *J* 6.9. 2.2 Hz, 6xCH), 1.08 (36H, d, *J* 6.9 Hz, 12xCH₃).

¹³C-NMR (CD₂Cl₂): d 146.1 (3xCₐᵣ-O), 141.4 (d, *J* 2.4 Hz, 6xCₐᵣ), 125.2 (3xCₐᵣH), 124.3 (6xCₐᵣH), 27.7 (d, *J* 7.8 Hz, 6xCH), 23.8 (12xCH₃).

HRMS (EI) [M]⁺: m/z berechnet: für C₃₆H₅₁O₃P 562.35703, gefunden: 562.35784.

### 10. Herstellung von rac-4,8-Di-tert-butyl-6-(2,4-di-tert-butylphenoxy)-1,2,10,11-tetramethyldibenzo[d,t][1,3,2]dioxa-phosphepin (Verbindung 10)

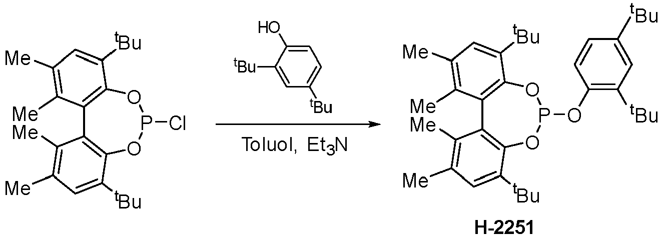

Eine Lösung der Chlor-Verbindung (355 mg, 0.80 mmol) und Triethylamin (121 mg, 1.20 mmol) in Toluol (5 ml) wird mit einer äquimolaren Menge von 2,4-Di-*tert*-butylphenol (165 mg) versetzt. Der Ansatz wurde 40 Stunden bei 100 °C gerührt, um nahezu vollständigen Umsatz zu erreichen. Nach Abkühlen und Filtration wurde das Lösemittel unter Vakuum entfernt und der Rückstand säulenchromatographisch gereinigt (Eluent: Cyclohexan/CH₂Cl₂ 99/1).

Ausbeute: 250 mg weißer Feststoff (51%), Smp. = 74-76 °C.

³¹P-NMR (CD₂Cl₂): d +134.7 ppm.

¹H-NMR (CD₂Cl₂): d 7.34 (1H, br d, J2.5 Hz, Hₐᵣ), 7.20 (1H, br s, Hₐᵣ), 7.18 (1H, br s, Hₐᵣ), 7.11 (1H, dd, *J* 8.4, 2.5 Hz, Hₐᵣ), 6.99 (1H, dd, *J* 8.4, 1.7 Hz, Hₐᵣ), 2.29 (3H, s, CH₃), 2.29 (3H, s, CH₃), 1.89 (3H, s, CH₃), 1.85 (3H, s, CH₃), 1.36 (9H, s, C(CH₃)₃), 1.35 (9H, s, C(CH₃)₃), 1.30 (9H, s, C(CH₃)₃), 1.23 (9H, s, C(CH₃)₃).

¹³C-NMR (CD₂Cl₂): d 149.4 (d, *J* 8.1 Hz, Cₐᵣ-O), 149.4 (Cₐᵣ-O), 145.7 (d, *J* 6.0 Hz, Cₐᵣ-O), 145.3 (Cₐᵣ), 139.5 (d, *J* 1.5 Hz, Cₐᵣ), 138.7 (d, *J* 3.1 Hz, Cₐᵣ), 137.8 (Cₐᵣ), 135.6 (Cₐᵣ), 134.7 (Cₐᵣ), 133.3 (Cₐᵣ), 132.6 (d, *J* 5.3 Hz, Cₐᵣ), 132.4 (Cₐᵣ), 130.9 (d, *J* 2.7 Hz, Cₐᵣ), 128.5 (CₐᵣH), 128.3 (CₐᵣH), 124.8 (CₐᵣH), 123.7 (CₐᵣH), 119.8 (d, *J* 21.5 Hz, CₐᵣH), 35.1 (^{t}C), 35.1 (^{t}C), 35.0 (^{t}C), 34.8 (^{t}C), 31.7 (3xCH₃), 31.6 (3xCH₃), 31.3 (d, *J* 5.1 Hz, 3xCH₃), 30.2 (3xCH₃), 20.6 (CH₃), 20.5 (CH₃), 16.9 (CH₃), 16.7 (CH₃).

HRMS (ESI-TOF) [M+H]⁺: *m*/*z* berechnet: für C₃₈H₅₃O₃P 589.3810, gefunden: 589.3821.

HRMS (ESI-TOF) [M+Na]⁺: *m*/*z* berechnet: für C₃₈H₅₃O₃P 611.3624, gefunden: 611.3639.

### 11. Herstellung von rac-4,8-Di-tert-butyl-1,2,10,11-tetramethyl-6-(naphthalen-1-yloxy)dibenzo[d,f][1,3,2]dioxa-phosphepin (Verbindung 11)

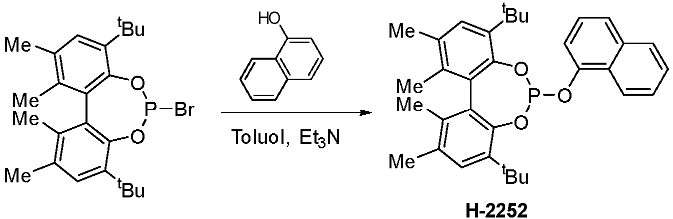

Eine Lösung des Phosphorbromids (278 mg, 0.60 mmol) in Toluol (5 ml) wird mit Triethylamin (91 mg, 0.90 mmol) versetzt und anschließend mit einer äquimolaren Menge von 1-Naphthol (87 mg) versetzt. Der Ansatz wurde über Nacht bei Raumtemperatur gerührt und anschließend vom ausgefallenen Niederschlag (Et₃NH⁺Br⁻) abfiltriert. Vom Filtrat wurde das Lösemittel unter Vakuum entfernt und der Rückstand säulenchromatographisch gereinigt (Eluent: Cyclohexan/CH₂Cl₂ 98/2).

Ausbeute: 250 mg weißer Feststoff (79%), Smp. = 70-72 °C.

³¹P-NMR (CD₂Cl₂): d +133.2 ppm.

¹H-NMR (CD₂Cl₂): d 8.03 (1H, m, Hₐᵣ), 7.84 (1H, m, Hₐᵣ), 7.63 (1H, br d, *J* 8.2 Hz, Hₐᵣ), 7.53-7.41 (2H, m, 2xHₐᵣ), 7.37 (1H, dd, *J* 7.9, 7.9 Hz, Hₐᵣ), 7.31 (1H, br s, Hₐᵣ), 7.20 (1H, br s, Hₐᵣ), 7.15 (1H, m, Hₐᵣ), 2.36 (3H, s, CH₃), 2.36 (3H, s, CH₃), 1.91 (3H, s, CH₃), 1.87 (3H, s, CH₃), 1.49 (9H, s, C(CH₃)₃), 1.31 (9H, s, C(CH₃)₃).

¹³C-NMR (CD₂Cl₂): d 148.4 (d, *J* 4.7 Hz, Cₐᵣ), 145.2 (d, *J* 6.4 Hz, Cₐᵣ), 144.8 (Cₐᵣ), 138.7 (d, *J* 3.1 Hz, Cₐᵣ), 137.0 (Cₐᵣ), 135.7 (d, *J* 1.5 Hz, Cₐᵣ), 135.3 (Cₐᵣ), 135.0 (Cₐᵣ), 133.5 (d, *J* 0.9 Hz, Cₐᵣ), 132.8 (Cₐᵣ), 132.4 (d, *J* 5.6 Hz, Cₐᵣ), 131.1 (d, *J* 3.1 Hz, Cₐᵣ), 129.4 (Cₐᵣ), 128.7 (d, *J* 1.2 Hz, CₐᵣH), 128.4 (CₐᵣH), 127.9 (CₐᵣH), 126.9 (CₐᵣH), 126.3 (CₐᵣH), 125.9 (CₐᵣH), 124.3 (d, *J* 1.4 Hz, CₐᵣH), 123.0 (CₐᵣH), 115.7 (d, *J* 12.6 Hz, CₐᵣH), 35.3 (^{t}C), 34.9 (^{t}C), 31.8 (3xCH₃), 31.3 (d, *J* 4.9 Hz, 3xCH₃), 20.6 (CH₃), 20.5 (CH₃), 16.9 (CH₃), 16.7 (CH₃).

HRMS (ESI-TOF) [M+H]⁺: *m*/*z* berechnet: für C₃₄H₃₉O₃P 527.2715, gefunden: 527.2720.

HRMS (ESI-TOF) [M+Na]⁺: *m*/*z* berechnet: für C₃₄H₃₉O₃P 549.2529, gefunden: 549.2538.

### 12. Herstellung von rac-4,8-Di-tert-butyl-6-((9,9-dimethyl-9H-xanthen-4-yl)oxy)-1,2,10,11-tetramethyldi-benzo[d,f][1,3,2]di-oxaphosphepin (Verbindung 12)

### Stufe 1: Herstellung von 9,9-Dimethyl-9H-xanthen-4-ol

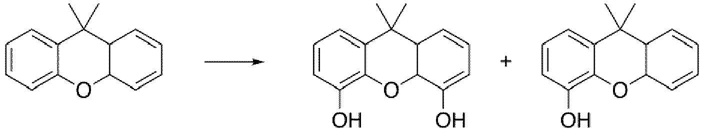

Zu einer Lösung des 9,9-Dimethyl-9H-xanthens (4.80 g, 22.8 mmol) und TMEDA (6.62 g, 57.0 mmol) in Ether (100 ml) wird bei -78 °C eine *n*-Butyllithium-Lösung in Hexan (2.5 M, 22.8 ml, 57.0 mmol) langsam hinzugegeben. Nach 20 Minuten lässt man auf Raumtemperatur erwärmen und rührt fünf Stunden weiter. Die Reaktionsmischung wird mit Triisopropylborat (17.15 g, 91.2 mmol) mittels einer Spritze versetzt und anschließend nach einer halben Stunde langsam mit einer wässrigen NaOH-Lösung (5.47 g, 136.8 mmol NaOH in 35 ml Wasser) hydrolysiert. Weitere 20 Minuten später fügt man Wasserstoffperoxid-Lösung (34%, 14 ml) hinzu und lässt über Nacht weiterrühren. Zur

Aufarbeitung werden die beiden Phasen getrennt und die wässrige Phase mit etwas Ether gewaschen. Durch Zugabe von Salzsäure (10%) wurde ein pH-Wert = 4 eingestellt und ein zäher Sirup fiel aus. Dieser wurde abgetrennt und getrocknet.

Bei der Reinigung durch Säulenchromatographie (Eluent: Cyclohexan/AcOEt 4/1) wurde neben dem eigentlichen Zielprodukt (Diol, 2.80 g) auch Anteile der Mono-Hydroxyverbindung erhalten.

Ausbeute: 530 mg weißer Feststoff (10%), Smp. = 92 °C.

¹H-NMR (DMSO-d₆): d 9.36 (1H, br s, OH), 7.50 (1H, m, Hₐᵣ), 7.23(1H, m, Hₐᵣ), 7.14-7.05 (2H, m, 2xHₐᵣ), 6.96-6.84 (2H, m, 2xHₐᵣ), 6.77 (1H, dd, *J* 7.4, 2.1 Hz, Hₐᵣ), 1.55 (6H, s, 2xCH₃).

¹³C-NMR (DMSO-d₆): d 149.7 (Cₐᵣ), 145.0 (Cₐᵣ), 138.4 (Cₐᵣ), 130.7 (Cₐᵣ), 129.8 (Cₐᵣ), 127.4 (CₐᵣH), 126.4 (CₐᵣH), 123.2 (CₐᵣH), 122.8 (CₐᵣH), 116.1 (CₐᵣH), 116.1 (CₐᵣH), 113.4 (CₐᵣH), 33.7 (^{t}C), 32.2 (2xCH₃).

HRMS (EI) [M]+: m/z berechnet: für C₁₅H₁₄O₂ 226.09883, gefunden 226.09832.

### Stufe 2: Herstellung von rac-4,8-Di-tert-butyl-6-((9,9-dimethyl-9H-xanthen-4-yl)oxy)-1,2,10,11-tetramethyldi-benzo[d,f][1,3,2]di-oxaphosphepin (Verbindung 12)

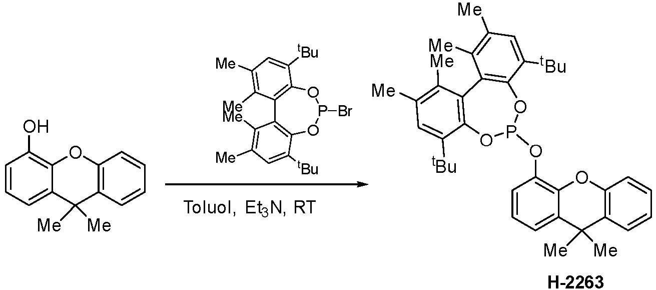

Zu einer Lösung des *rac*-6-Bromo-4,8-di-*tert*-butyl-1,2,10,11-tetramethyldibenzo[d,tJ[1,3,2]dioxa-phosphepins (417 mg, 0.90 mmol) in Toluol (4 ml) werden bei Raumtemperatur zunächst Triethylamin (137 mg, 1.35 mmol) und anschließend 9,9-Dimethyl-9H-xanthen-4-ol (204 mg, 0.90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 190 mg weißer Feststoff (35%), Smp. = 90-92 °C.

³¹P-NMR (CD₂Cl₂): d +132.7 ppm.

¹H-NMR (CD₂Cl₂): d 7.43 (1H; dd, *J* 8.3, 1.9 Hz, Hₐᵣ), 7.30 (1H, br s, Hₐᵣ), 7.23-7.08 (5H, m, 5xHₐᵣ), 6.89 (1H, t, *J* 8.0 Hz, Hₐᵣ), 6.60 (1H, dt, *J* 8.0, 1.5 Hz, Hₐᵣ), 2.33 (3H, s, CH₃), 2.27 (3H, s, CH₃), 1.86 (3H, s, CH₃), 1.85 (3H, s, CH₃), 1.62 (3H, s, CH₃), 1.61 (3H, s, CH₃), 1.54 (9H, s, C(CH₃)₃), 1.31 (9H, s, C(CH₃)₃).

¹³C-NMR (CD₂Cl₂): d 150.2 (2xCₐᵣ), 145.0 (d, *J* 5.8 Hz, Cₐᵣ), 145.0 (Cₐᵣ), 145.0 (Cₐᵣ), 142.2 (d, *J* 2.7 Hz, Cₐᵣ), 138.7 (d, *J* 3.0 Hz, Cₐᵣ), 138.1 (Cₐᵣ), 135.5 (Cₐᵣ), 135.0 (Cₐᵣ), 133.2 (Cₐᵣ), 132.6 (Cₐᵣ), 132.5 (d, *J* 5.3 Hz, Cₐᵣ), 132.1 (Cₐᵣ), 130.2 (Cₐᵣ), 128.7 (CₐᵣH), 128.2 (CₐᵣH), 127.7 (CₐᵣH), 126.5 (CₐᵣH), 123.8 (CₐᵣH), 122.7 (CₐᵣH), 121.9 (CₐᵣH), 120.6 (d, *J* 5.3 Hz, CₐᵣH), 117.2 (CₐᵣH), 35.3 (^{t}C), 34.9 (^{t}C), 34.5 (^{t}C), 33.0 (CH₃), 32.4 (CH₃), 31.8 (3xCH₃), 31.2 (d, *J* 5.0 Hz, 3xCH₃), 20.5 (CH₃), 20.5 (CH₃), 16.8 (CH₃), 16.6 (CH₃).

HRMS (ESI-TOF) [M+H]⁺: *m*/*z* berechnet: für C₃₉H₄₅O₄P 609.3134, gefunden: 609.3135.

HRMS (ESI-TOF) [M+Na]⁺: *m*/*z* berechnet: für C₃₉H₄₅O₄P 631.2947, gefunden: 631.2956.

### 13. Herstellung von rac-4,8-Di-tert-butyl-6-((2,7-di-tert-butyl-9,9-dimethyl-9H-xanthen-4-yl)oxy)-1,2,10,11-tetramethyl-dibenzo[d,f][1,3,2]dioxaphosphepin (Verbindung 13)

### Stufe 1: Herstellung von 2,7-Di-tert-butyl-9,9-dimethyl-9H-xanthen-4-ol

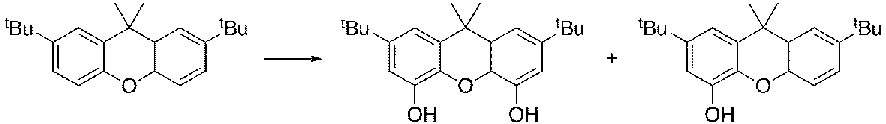

Zu einer Lösung des 2,7-Di-*tert*-butyl-9,9-dimethyl-9*H*-xanthens (4.78 g, 14.8 mmol) und TMEDA (4.29 g, 37.0 mmol) in Ether (75 ml) wird bei -78 °C eine n-Butyllithium-Lösung in Hexan (2.5 M, 14.8 ml, 37.0 mmol) langsam hinzugegeben. Nach 20 Minuten lässt man auf Raumtemperatur erwärmen und rührt fünf Stunden weiter. Die Reaktionsmischung wird mit Triisopropylborat (11.15 g, 59.3 mmol) mittels einer Spritze versetzt und anschließend nach einer halben Stunde langsam mit einer wässrigen NaOH-Lösung (3.55 g, 88.8 mmol NaOH in 23 ml Wasser) hydrolysiert. Weitere 20 Minuten später fügt man Wasserstoffperoxid-Lösung (34%, 9 ml) hinzu und lässt über Nacht weiterrühren. Zur Aufarbeitung werden die beiden Phasen getrennt. Entgegen der Originalvorschrift für die 2,7-Dimethyl-Verbindung verbleibt das Produkt in der Ether-Phase. Diese wird mit Wasser (50 ml) versetzt und gewaschen bis nach der sukzessiven Zugabe von HCl der pH-Wert auf 4-5 eingestellt ist. Nach der Phasentrennung wird die Etherphase getrocknet (Na₂SO₄) und filtriert. Der Ether wird entfernt und der Rückstand durch Säulenchromatographie (Eluent: Cyclohexan/AcOEt 19/1) gereinigt. Neben der eigentlichen Zielverbindung (Diol, 2.80 g) wurden auch Anteile der Mono-Hydroxyverbindung isoliert.

Ausbeute: 780 mg weißer Feststoff (15%), Smp. = 116-118 °C

¹H-NMR (Aceton-d₆): d 8.00 (1H, br s, OH), 7.54 **(1H,** d, *J* 2.4 Hz, Hₐᵣ), 7.25 (1H, dd, *J* 8.5, 2.4 Hz, Hₐᵣ), 7.03 (1H, s, Hₐᵣ), 7.01 (1H, d, *J* 6.0 Hz, Hₐᵣ), 6.87 (1H, d, *J* 2.3 Hz, Hₐᵣ), 1.65 (6H, s, 2xCH₃), 1.33 (9H, s, C(CH₃)₃), 1.31 (9H, s, C(CH₃)₃).

¹³C-NMR (Aceton-d₆): d 148.8 (Cₐᵣ), 146.3 (2xCₐᵣ), 145.1 (Cₐᵣ), 137.2 (Cₐᵣ), 130.6 (Cₐᵣ), 130.1 (Cₐᵣ), 125.1 (CₐᵣH), 123.7 (CₐᵣH), 116.4 (CₐᵣH), 113.8 (CₐᵣH), 111.8 (CₐᵣH), 35.2 (^{t}C), 35.0 (2x^{t}C), 32.7 (2xCH₃), 31.8 (3xCH₃), 31.8 (3xCH₃).

HRMS (EI) [M]⁺: *m*/*z* berechnet: für C₂₃H₃₀O₂ 338.22403, gefunden 338.22336.

### Stufe 2: Herstellung von rac-4,8-Di-tert-butyl-6-((2,7-di-tert-butyl-9,9-dimethyl-9H-xanthen-4-yl)oxy)-1,2,10,11-tetramethyl-dibenzo[d,f][1,3,2]dioxaphosphepin

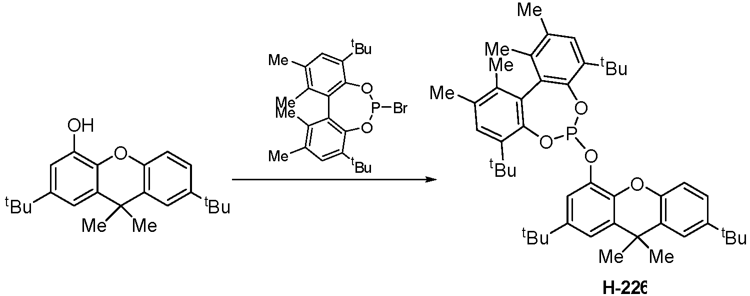

Zu einer Lösung des *rac*-6-Bromo-4,8-di-*tert*-butyl-1,2,10,11-tetramethyldibenzo[*d*,*f*][1,3,2]dioxa-phosphepins (417 mg, 0.90 mmol) in Toluol (4 ml) werden bei Raumtemperatur zunächst Triethylamin (137 mg, 1.35 mmol) und anschließend 2,7-Di-tert-butyl-9,9-dimethyl-9H-xanthen-4-ol (305 mg, 0.90 mmol) gegeben. Nach dem Rühren über Nacht wird vom ausgefallenen Niederschlag abfiltriert und das Filtrat im Vakuum eingeengt. Der verbleibende Rückstand wird durch Säulenchromatographie (Eluent: Cyclohexan/AcOEt 99/1) gereinigt.

Ausbeute: 480 mg weißer Feststoff (74%), Smp. = 100-102 °C.

³¹P-NMR (CD₂Cl₂): d +131.8 ppm.

¹H-NMR (CD₂Cl₂): d 7.42 (1H, d, *J* 2.3 Hz, Hₐᵣ), 7.31 (1H, s, Hₐᵣ), 7.24 (1H, dd, *J* 8.5, 2.3 Hz, Hₐᵣ), 7.17 (1H, s, Hₐᵣ), 7.16 (1H, dd, *J* 2.3, 0.9 Hz, Hₐᵣ), 7.03 (1H, d, *J* 8.5 Hz, Hₐᵣ), 6.73 (1H, dd, *J* 2.3,

1.5 Hz, Hₐᵣ), 2.31 (3H, s, CH₃), 2.27 (3H, s, CH₃), 1.86 (3H, s, CH₃), 1.85 (3H, s, CH₃), 1.64 (3H, s, CH₃), 1.62 (3H, s, CH₃), 1.57 (9H, s, C(CH₃)₃), 1.34 (9H, s, C(CH₃)₃), 1.33 (9H, s, C(CH₃)₃), 1.24 (9H, s, C(CH₃)₃).

¹³C-NMR (CD₂Cl₂): d 148.1 (Cₐᵣ), 146.3 8Cₐᵣ), 145.7 (Cₐᵣ), 145.2 (d, *J* 6.3 Hz, Cₐᵣ), 145.1 8Cₐᵣ), 140.1 (d, *J* 2.9 Hz, Cₐᵣ), 139.1 Cₐᵣ), 138.7 (d, *J* 2.9 Hz, Cₐᵣ), 138.2 (Cₐᵣ), 135.5 (Cₐᵣ), 134.9 (Cₐᵣ), 133.3 (Cₐᵣ), 132.6 (Cₐᵣ), 132.4 (d, *J* 5.8 Hz, Cₐᵣ), 131.3 (d, *J* 2.6 Hz, Cₐᵣ), 131.0 (Cₐᵣ),129.3 (Cₐᵣ), 128.9 (CₐᵣH), 128.2 (CₐᵣH), 124.8 (CₐᵣH), 123.2 (CₐᵣH), 118.6 (CₐᵣH), 118.5 (d, *J* 4.6 Hz, CₐᵣH), 116.4 (CₐᵣH), 35.5 (^{t}C), 35.0 (2x^{t}C), 34.8 (2x^{t}c), 33.2 (CH₃), 32.5 (CH₃), 31.9 (3xCH₃), 31.7 (3xCH₃), 31.5 (3xCH₃), 31.3 (d, *J* 5.0 Hz, 3xCH₃), 20.6 (CH₃), 20.5 (CH₃), 16.9 (CH₃), 16.7 (CH₃).

HRMS (ESI-TOF) [M+H]⁺: *m*/*z* berechnet: für C₄₇H₆₁O₄P 721.4385, gefunden: 721.4386.

HRMS (ESI-TOF) [M+Na]⁺: *m*/*z* berechnet: für C₄₇H₆₁O₄P 743.4199, gefunden: 743.4207.

### 14. Durchführung von Katalyse-Versuchen mit den Liganden gemäß Verbindungen 1 bis 13

### a) Durchführung:

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Cis/trans-2-Penten (Aldrich) wurde über Natrium am Rückfluss erhitzt und unter Argon destilliert. Im Autoklav wurden unter Argonatmosphäre Lösungen der Katalysatorvorstufe und des Liganden jeweils in Toluol gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischung wurden entnommen, mit 10 ml Pentan verdünnt und gaschromatographisch analysiert: HP 5890 Series II plus, PONA, 50 m x 0,2 mm x 0,5 µm.

### b) Ergebnisse in der Hydroformylierung von 2-Penten:

Für die Verbindungen 1 bis 13 wurden die folgenden Umsätze und n-Selektivitäten bestimmt, wobei n-Selektivität die Selektivität für die Bildung linearer Aldehyde angibt und aus dem Verhältnis n-Aldehyd : i-Aldehyd ermittelt werden kann.

| **Verbindung** | **Umsatz (%)** |
|---|---|
| 1 | 99 |
| 1^{a} | >99 |
| 2 | 99 |
| 3 | 1 |
| 4 | 92 |
| 5 | 99 |
| 5^{a} | 99 |
| 6 | >99 |
| 7 | <1 |
| 8 | >99 |
| 9 | 6 |
| 10 | 98 |
| 11 | 99 |
| 12 | 99 |
| 13 | 99 |

Standardbedingungen: Substrat: 2-Penten, T: 120 °C, *t:* 4 h, 20 bar CO/H₂ (1:1), Solvens Toluol, 100 ppm Rh, [Olefin]= 2,41 M, Rh/Ligand 1/5;
^{a} Rh/Ligand = 1/3

## Patentansprüche

1. Verbindung gemäß allgemeiner Formel (I) 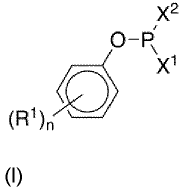 wobei,
- R¹, unabhängig voneinander, einen Propylrest, insbesondere *iso*-Propylrest, einen Butylrest, insbesondere *tert*-Butylrest, oder einen Methylrest bezeichnet, jedoch mit der Maßgabe, dass mindestens ein Rest R¹ einen Propylrest, insbesondere *iso-*Propylrest, oder einen Butylrest, insbesondere *tert*-Butylrest, bezeichnet,
- n eine ganze Zahl 1, 2 oder 3 darstellt,
- X¹ und X², gleich oder verschieden und/oder unabhängig voneinander, jeweils eine gegebenenfalls substituierte Phenylgruppe oder einen Rest O-R² bezeichnet, wobei R² einen organischen Rest, insbesondere aromatischen organischen Rest, bezeichnet, wobei X¹ und X² gegebenenfalls miteinander verbunden und/oder verbrückt sein können.

2. Verbindung nach Anspruch 1,
wobei R¹ ausgewählt ist aus einem der folgenden Reste: 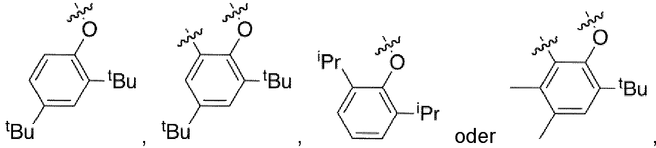 insbesondere

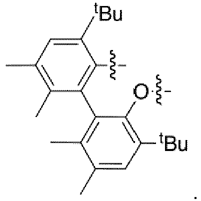

3. Verbindung nach Anspruch 1 oder 2,
wobei X¹ und X², gleich oder verschieden und/oder unabhängig voneinander, jeweils ausgewählt sind aus einem der folgenden Reste

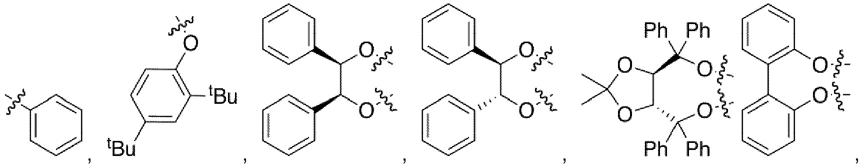

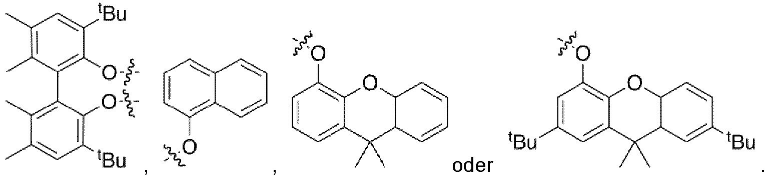

4. Verbindung nach einem der vorangehenden Ansprüche,
wobei die Verbindung gemäß Formel (I) ausgewählt ist aus einer der folgenden Verbindungen

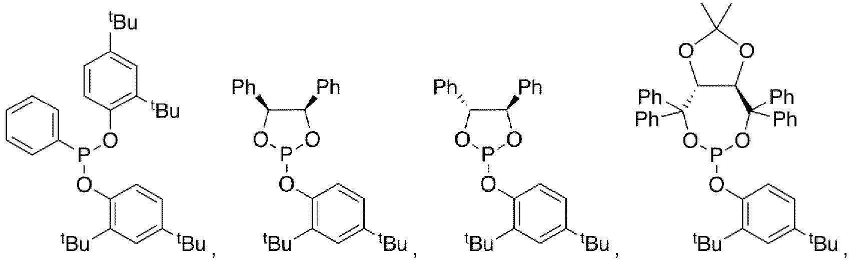

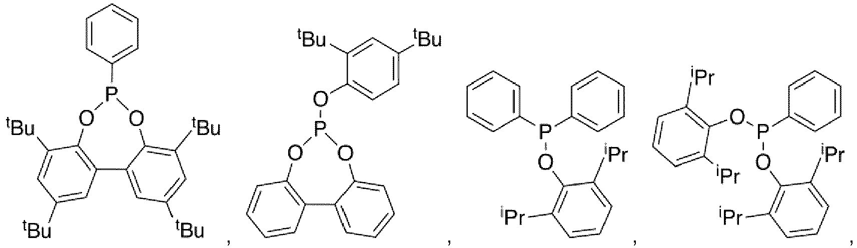

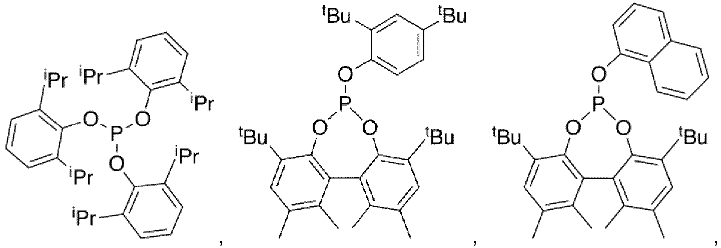

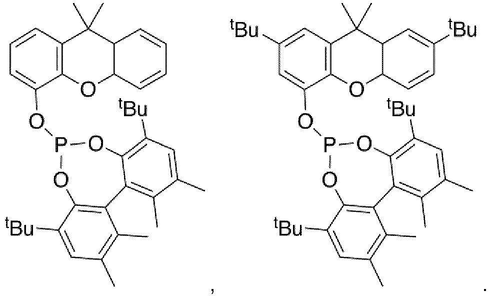

5. Verfahren zur Herstellung einer Verbindung gemäß Formel (I),
wobei eine reaktive Phosphor(III)spezies, insbesondere ausgewählt aus Phosphor(III)halogenid oder Phosphor(III)amidit, umgesetzt wird mit einem Hydroxylgruppen-haltigen Reagenz, so dass eine Verbindung gemäß Formel (I) resultiert.

6. Verfahren nach Anspruch 5,
wobei das Verfahren bei einer Temperatur im Bereich von 10 °C bis 120 °C und/oder in einem, bevorzugt organischen, insbesondere aprotischen, Lösemittel durchgeführt wird.

7. Verfahren nach Anspruch 5 oder 6,
wobei das Verfahren in Gegenwart einer Base, insbesondere eines Amins, vorzugsweise Triethylamin, durchgeführt wird.

8. Katalytisch aktive Verbindung,
wobei die katalytisch aktive Verbindung mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, zusammen mit mindestens einer Verbindung gemäß Formel (I) als Ligand umfasst.

9. Verwendung einer Verbindung nach einem der Ansprüche 1 bis 4 oder einer katalytisch aktiven Verbindung nach Anspruch 8 in einem Hydroformylierungsverfahren, insbesondere zur Umsetzung ethylenisch ungesättigter Verbindungen zu den entsprechenden Aldehyden.

10. Verfahren zur Hydroformylierung,
wobei mindestens eine ethylenisch ungesättigte Verbindung in Gegenwart einer Verbindung nach einem der Ansprüche 1 bis 4 oder einer katalytisch aktiven Verbindung nach Anspruch 8 einer Hydroformylierung unterzogen wird, insbesondere zu dem entsprechenden Aldehyd umgesetzt wird.

11. Verwendung nach Anspruch 9 oder Verfahren nach Anspruch 10,
wobei das Hydroformylierungsverfahren die Verfahrensschritte
a) Vorlegen einer ethylenisch ungesättigten Verbindung;
b) Zugabe einer Verbindung gemäß einem der Ansprüche 1 bis 4 und einer Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, oder einer katalytisch aktiven Verbindung nach Anspruch 8;
c) Zuführen von H₂ und CO,
d) Erwärmen des Reaktionsgemisches aus a) bis c), wobei das Olefin zu einem Aldehyd umgesetzt wird,
umfasst.

12. Verwendung oder Verfahren nach einem der vorangehenden Ansprüche,
wobei die ethylenisch ungesättigte Verbindung in Verfahrensschritt a) ausgewählt ist aus: Ethen, Propen, 1-Buten, *cis-* und/oder *trans*-2-Buten, *iso*-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

13. Verwendung oder Verfahren nach einem der vorangehenden Ansprüche,
wobei die Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, ausgewählt ist aus: Rh(acac)(CO)₂, [(acac)Rh(COD)], wobei acac = Acetylacetonat-Anion und COD = 1,5-Cyclooctadien) sind, und/oder Rh₄CO₁₂, oder
wobei die katalytisch aktive Verbindung neben der mindestens einen Verbindung gemäß Formel (I) mindestens einen weiteren Liganden ausgewählt aus (acac), (CO) und/oder (COD) umfasst.

14. Verwendung oder Verfahren nach einem der vorangehenden Ansprüche,
wobei das Zuführen von CO in Verfahrensschritt c) mit einem Druck in dem Bereich von 1 bis 6 MPa (10 bis 60 bar) erfolgt.

15. Verwendung oder Verfahren nach einem der vorangehenden Ansprüche,
wobei das Erwärmen des Reaktionsgemisches in Verfahrensschritt d) auf eine Temperatur in dem Bereich von 80 °C bis 160 °C erfolgt.
